# EUROPÄISCHE PATENTANMELDUNG

(11) **EP 2 784 058 A1**
(43) Veröffentlichungstag der Anmeldung: **01.10.2014**
(21) Anmeldenummer: 13180588.9
(22) Anmeldetag: 15.08.2013
(51) Int. Cl.: C07C 213/10, C07C 215/08, B65D 85/84

(54) **Verfahren zur Verbesserung der Farbstabilität eines Ethanolamins**

(71) Anmelder: BASF SE, 67056 Ludwigshafen (DE)
(72) Erfinder: Grünanger, Christian, 68163 Mannheim (DE); Schmitt, Markus, 69115 Heidelberg (DE); Bou Chedid, Roland, 68159 Mannheim (DE); Melder, Johann-Peter, 67459 Böhl-Iggelheim (DE)

(57) **Zusammenfassung**

Verfahren zur Verbesserung der Farbstabilität eines Ethanolamins, wobei das Ethanolamin in einem aus Kunststoff gefertigten Behälter gelagert wird.

## Beschreibung

Die vorliegende Erfindung betrifft ein Verfahren Verbesserung der Farbstabilität eines Etha-nolamins.

Ethanolamine sind wichtige Zwischenprodukte für die chemische und pharmazeutische Industrie. Dimethylethanolamin (DMEOA) findet z. B. in Form von Salzen, Seifen, Ether und Ester als Emulgator und oberflächenaktive Substanz sowie als Katalysator in der Polyurethanchemie Anwendung auf den verschiedensten Gebieten. In der pharmazeutischen Industrie wird es zur Synthese von Wirkstoffen (Tranquilizer, Antihistaminika und Analgetika) benutzt. Verfärbungen in Ethanolaminen sind bei den meisten Anwendungen unerwünscht.

Es wird angenommen, dass die unerwünschte Bildung von Verfärbungen in Ethanolaminen wie z.B. DMEOA auf komplizierte Zersetzungsmechanismen (E. Tobler et al., Helv. Chim. Acta 52, 1969, Seite 408 - 418) von auftretenden quartären Ammoniumverbindungen zu flüchtigen ungesättigten Verbindungen, welche dann polymerisieren, zurückzuführen ist (DD 203 534 A).

Der Stand der Technik beschreibt Verfahren zur Verbesserung der Farbqualität von Ethanola-minen, in denen
a) die Bildung der farbgebenden Nebenkomponenten durch geeignete Herstell-Fahrweise weitgehend unterdrückt wird und oder
b) in denen die farbgebenden Nebenkomponenten durch spezielle Verfahren effektiv destillativ abgetrennt werden und oder
c) in denen die farbgebenden Nebenkomponenten in einem separaten Reaktionsschritt chemisch umgewandelt werden und oder
d) in denen nach der destillativen Aufreinigung Additive zugesetzt werden, die die Farbbildung verhindern.

Beispielhaft für die Verfahrensweise a) sei auch US 2012/0157715 A1 (BASF SE) genannt, in der Reaktionstemperatur und Druck, Verweilzeit im Reaktor, sowie eine thermische Nachbehandlung des Reaktionsaustrages beschrieben sind. In dieser Patentanmeldung wird auch weiterer Stand der Technik für die Verfahrensweisen b) bis d) angegeben.

Die bekannten Fahrweisen der Ethanolamine-Produktion ausgehend von Ethylenoxid (EO) als einem der Synthesebausteine, z.B. der Dimethylethanolamin-Produktion aus Dimethylamin und EO, kennzeichnen sich dadurch aus, dass mehr oder weniger Nebenkomponenten gebildet werden, die zunächst farblos sind, aber im Laufe der Zeit in farbgebende Moleküle umgewandelt werden können.

Solche problematischen Nebenkomponenten werden, wie erfindungsgemäß erkannt wurde, z.B. vermehrt gebildet, wenn das betreffende Ethanolamin unter Hochlast produziert wird. Unter Hochlastproduktion ist hier zu verstehen, dass das betreffende Ethanolamin bei seiner Herstellung in dem bzw. den Synthesereaktoren insgesamt einer Verweilzeit von ≤ 5 Min., z.B. 1 bis 5 Min., besonders ≤ 4 Min., unterliegt.

Folgende Literaturstellen beschäftigen sich mit der Kompatibilität von DMEOA bzw. Dialkyl-ethanolaminen mit Kunststoffen, jedoch nicht mit der Verbesserung der Farbstabilität von Etha-nolaminen:

FR 2 938 453 A1 (P. Broutin et al.) beschreibt eine Methode, um die oxidative Zersetzung einer Absorber-Lösung (enthält z. B. Dimethylethanolamin) für die Gaswäsche zu verringern. Gelehrt wird eine spezielle Stahllegierungen oder einen Überzug des Stahls mit Emaille oder Kunststoff.

US 2002/0120167 A1 (Nippon Shokubai Co., Ltd.) beschreibt die Darstellung von hochreinen Alkanolaminen. Nach der Destillationskolonne bis zum Lagertank ist der Stahl mit Kunststoff überdeckt. Die Patente beschreiben einen spezifischen Fe-Gehalt, jedoch keine Farbzahl.

WO 2010/149936 A1 (Arkema France) beschreibt die Darstellung von Alkylalkanolaminen aus Carbonylverbindungen und Hydroxylalkylamin. Dort wird auch von einer Lagerung in Glas, HDPE und Stahl berichtet, wobei die Lagerungszeit in Glas und HDPE länger angegeben ist. (Vgl. dort auch Absatz [0038]).

Das Federal Register of Packaging des United States Dept. Of Transportation, Vol. 55. No. 246, 21.12.1990, beschreibt Packmittelauthorisierungen, unter zahlreichen Materialen wie Stahl, Aluminium, Glas, Holz, auch "Plastic", ohne jedoch auf Vor- oder Nachteile in Bezug auf die Farbstabilität von Ethanolaminen einzugehen.

Der vorliegenden Erfindung lag die Aufgabe zugrunde, unter Überwindung von einem oder mehreren Nachteilen des Stands der Technik, ein wirtschaftliches Verfahren zur Verbesserung der Farbstabilität von Ethanolaminen, darunter auch N,N-Dialkyl-ethanolaminen, insb. DMEOA, aufzufinden. Das Verfahren sollte unter betriebssicheren Arbeitsbedingungen die Farbstabilität von Ethanolaminen erhöhen. Das Verfahren sollte dabei vorteilhafterweise ohne den Einsatz zusätzlicher Hilfsstoffe (stabilisierende Zusätze, die die Verfärbung inhibieren) auskommen.

Demgemäß wurde ein Verfahren zur Verbesserung der Farbstabilität eines Ethanolamins gefunden, welches dadurch gekennzeichnet ist, dass das Ethanolamin in einem aus Kunststoff gefertigten Behälter gelagert wird.

Die Lagerung erfolgt in dem aus Kunststoff gefertigten Behälter bevorzugt in Abwesenheit von Sauerstoff (O₂). Dies kann dadurch erreicht werden, dass das Behältervolumen vollständig mit dem Ethanolamin ausgefüllt ist oder, wenn dies nicht der Fall ist, das Differenzvolumen mit einem Schutzgas ausgefüllt ist. Als Schutzgas eignet sich z.B. Stickstoff oder ein Edelgas wie Argon. Unter "Abwesenheit von Sauerstoff (O₂)" ist in diesem Sinne zu verstehen, dass die Schutzgasatmospäre einen O₂-Restgehalt von kleiner 5 Vol.-%, besonders im Bereich von 0 bis 3 Vol.-% aufweist.

Der Behälter weist für das Ethanolamin und ggf. das Schutzgas bevorzugt ein Volumen im Bereich von 0,5 bis 2000 Liter, besonders im Bereich von 20 bis 400 Liter, weiter besonders im Bereich von 100 bis 300 Liter, auf.

Bei dem Behälter handelt es sich besonders um ein Kunststofffass oder einen Kunststofftank.

Weiter besonders stellt der aus Kunststoff gefertigte Behälter die Innenauskleidung eines Stahl-fasses oder eines Stahltanks dar.

Bevorzugt handelt es sich bei dem Kunststoff um ein Polyethylen (PE), Polypropylen (PP), Polystyrol (PS), Polyurethan (PU), Polytetrafluorethen (PTFE, Teflon®), Polyketon, Polyamid, Epoxy-Harz, Epoxy-Amin-Harz, Phenolharz, einen Polyester, Polyvinylester oder Polyvinylether.

Bei dem Polyketon handelt es sich beispielsweise um ein Copolymer aus Kohlenmonoxid und Ethylen.

Bei dem Phenolharz handelt es sich beispielsweise um das Produkt einer Polykondensation eines Aldehyds mit Phenol.

Bei dem Polyamid handelt es sich beispielsweise um das Produkt einer Polykondensation einer Dicarbonsäure mit einem Diamin.

Bei dem Epoxy-Harz handelt es sich beispielsweise um das Produkt einer Umsetzung von Epichlorhydrin mit einem Diol, Polyol oder einer Dicarbonsäure.

Bei dem Epoxy-Amin-Harz handelt es sich beispielsweise um das Produkt einer Umsetzung von Epichlorhydrin mit einem Diamin.

Bei dem Polyester handelt es sich beispielsweise um das Produkt einer Polykondensation einer Dicarbonsäure mit einem Diol, insbesondere auch um Polyalkylenterephthalate, wie Polyethylenterephthalat (PET), Polybutylenterephthalat (PBT) und Poly(1,4-cyclohexandimethylenterephthalat) (PCDT).

Bei dem Polyvinylester handelt es sich beispielsweise um das Produkt einer Polymerisation eines Vinylesters.

Bei dem Polyvinylether handelt es sich beispielsweise um das Produkt einer Polymerisation eines Vinylethers.

Besonders bevorzugt handelt es sich bei dem Polyethylen-Kunststoff um ein HD-PE (High-Density-PE), LLD-PE (Linear-Low-Density-PE) oder LD-PE (Low-Density-PE). (Kurzzeichen nach DIN EN ISO 1043-1: 2002-06; vgl. Römpp Online, Version 3.12, G. Thieme Verlag, 2013, Dokumentkennungen RD-08-00629, RD-12-01405 und RD-12-00585).

Beispiel für ein ganz besonders bevorzugtes HD-PE ist Lupolen 5261 Z, erhältlich von lyondell-basell (www.lyondellbasell.com).

Das erfindungsgemäße Verfahren eignet sich besonders zur Herstellung eines Ethanolamins mit einer APHA-Farbzahl ≤ 35 nach einer Lagerzeit von 25 Wochen bei 20°C, wobei die APHA-Farbzahl zu Beginn ≤ 15, besonders ≤ 10, betrug.
Ganz besonders eignet es sich zur Herstellung eines Ethanolamins mit einer APHA-Farbzahl ≤ 30 nach einer Lagerzeit von 25 Wochen bei 20°C, wobei die APHA-Farbzahl zu Beginn ≤ 15, besonders ≤ 10, betrug.

Bei dem Ethanolamin handelt es sich besonders um Monoethanolamin (MEOA), Diethanolamin (DEOA), Triethanolamin (TEOA) oder ein N-Alkyl-diethanolamin der Formel I 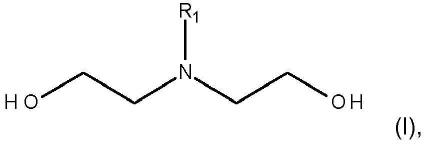 wobei R¹ eine C₁- bis C₈-Alkylgruppe, besonders eine C₁- bis C₄-Alkylgruppe, bedeutet.

Insbesondere handelt es sich bei dem Ethanolamin um ein N,N-Dialkyl-ethanolamin der Formel **II** 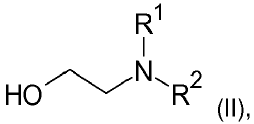 wobei R¹ und R² unabhängig voneinander eine C₁- bis C₈-Alkylgruppe, besonders R¹ und R² unabhängig voneinander eine C₁- bis C₄-Alkylgruppe, bedeuten.

Weiter besonders handelt es sich bei dem Ethanolamin um die Verbindung N,N-Dimethyl-ethanolamin (DMEOA), insbesondere hergestellt durch Umsetzung von EO mit Dimethylamin (DMA).

R¹ im Alkanolamin I und R¹ und R² im Alkanonamin II bedeuten (R¹ und R² unabhängig voneinander) eine lineare, cyclische und/oder verzweigte C₁- bis C₈-Alkylgruppe, bevorzugt C₁- bis C₄-Alkylgruppe. Beispiele für solche Alkylgruppen sind Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, Cyclopentyl, n-Hexyl, iso-Hexyl, sec.-Hexyl, Cyclohexyl, Cyclopentylmethyl, n-Heptyl, iso-Heptyl, Cyclohexylmethyl, n-Octyl, iso-Octyl, 2-Ethylhexyl. Besonders bevorzugt sind Methyl und Ethyl.

Das im erfindungsgemäßen Verfahren eingesetzte Ethanolamin wurde zuvor in einem oder mehreren Reaktoren bevorzugt durch Umsetzung von Ethylenoxid (EO) mit Ammoniak [z.B. gemäß EP 673 920 A2, EP 1 132 371 A1, EP 1 291 339 A2, WO 2001/94290 A1 (alle BASF AG), vgl. auch K. Weissermel, H.-J. Arpe, Industrielle Organische Chemie, 5. Auflage 1998, Kap. 7.2.3, Seite 174-175] oder einem entsprechenden Monoalkylamin (R¹NH₂) [z.B. gemäß EP 70978 A1 (Pennwalt Corp.)] oder einem entsprechenden Dialkylamin (R¹R²NH) [z.B. gemäß US 2012/0157715 A1 (BASF SE)] hergestellt (synthetisiert).

Bevorzugt erfolgt die Herstellung des im erfindungsgemäßen Verfahren eingesetzten N,N-Dialkyl-ethanolamins der Formel II, z.B. DMEOA, durch Umsetzung von Ethylenoxid (EO) mit einem entsprechenden Dialkylamin (R¹R²NH) in Gegenwart von Wasser, wobei die Umsetzung in einem Reaktor kontinuierlich erfolgt, die Reaktionstemperatur im Bereich von 90 bis 180 °C und die Verweilzeit (VWZ) im Reaktor im Bereich von 1 bis 8 Min. liegt, der Reaktoraustrag bei einer Temperatur im Bereich von 80 bis 160 °C über eine Zeit im Bereich von 20 bis 1000 Min. thermisch behandelt und danach das N,N-Dialkyl-ethanolamin destillativ abgetrennt wird. Ein solches Verfahren ist in US 2012/0157715 A1 (BASF SE) beschrieben.

Die Umsetzung erfolgt bevorzugt in Gegenwart von 2,5 bis 50 Gew.-%, besonders bevorzugt 5 bis 35 Gew.-%, weiter bevorzugt 10 bis 30 Gew.-%, Wasser (bezogen auf das Reaktionsgemisch).

Bevorzugt werden die Edukte in einem molaren Verhältnis von Dialkylamin : EO im Bereich von 1,1 bis 10, besonders bevorzugt 1,5 bis 9, weiter bevorzugt 3 bis 8, eingesetzt.

Bevorzugt erfolgt die Umsetzung in einem flüssigkeitsgekühlten Rohrreaktor. Besonders bevorzugt ist ein Doppelmantelreaktor. Der Reaktor kann im Gleich- oder Gegenstrom gekühlt werden. Bezüglich der Produktqualität (Farbzahl, Farbstabilität) hat sich die Gleichstromkühlung als vorteilhaft erwiesen. Daher fließt weiter bevorzugt die Kühlflüssigkeit im Gleichstrom durch den Doppelmantelreaktor.

Die Umsetzung erfolgt bevorzugt bei einem Absolutdruck im Bereich von 10 bis 40 bar, besonders bevorzugt 15 bis 30 bar.

Die Reaktionstemperatur liegt bevorzugt im Bereich von 110 bis 170 °C, besonders bevorzugt im Bereich von 125 bis 160 °C.

Die VWZ im Reaktor liegt bevorzugt im Bereich von 1,5 bis 7 Min., bevorzugt 2 bis 5 Min., weiter bevorzugt im Bereich von 2 bis 4 Min..

Bevorzugt wird der Reaktoraustrag bei einer Temperatur im Bereich von 80 bis 160 °C, besonders 125 bis 160 °C, bevorzugt 130 bis 155°C, über eine Zeit im Bereich von 20 bis 1000 Min., bevorzugt 30 bis 700 Min., weiter besonders 70 bis 190 Min., thermisch behandelt.

Bevorzugt wird vor der thermischen Behandlung des Reaktoraustrags unumgesetztes Dial-kylamin destillativ abgetrennt.
Die thermische Behandlung kann in einem separaten Behälter (Verweilzeitbehälter) oder bevorzugt im Sumpfbehälter der Dialkylamin-Destillationskolonne erfolgen.

Nach der thermischen Behandlung erfolgt eine destillative Aufarbeitung zur Abtrennung des N,N-Dialkyl-ethanolamins.
Nach destillativer Abtrennung und Rückführung von überschüssigem Dialkylamin wird das Wasser abgetrennt und bevorzugt in das Verfahren zurückgeführt. Die Reindestillation des N,N-Dialkyl-ethanolamins ist diskontinuierlich möglich, wird aber bevorzugt kontinuierlich durchgeführt. In der Reindestillation werden Hochsieder (z. B. höher ethoxylierte Produkte und Vinyloxyethanol) als Bodenprodukt (Sumpfprodukt) ausgeschleust, das N,N-Dialkyl-ethanolamin, z. B. das DMEOA, wird entweder über Kopf oder bevorzugt über einen Seitenabzug abdestilliert. Leichtsieder wie Alkoxyethanol können in diesem Fall über Kopf abgetrennt werden. Besonders bevorzugt erfolgt also die destillative N,N-Dialkyl-ethanolamin -Abtrennung kontinuierlich in einer Seitenabzugskolonne, in der das N,N-Dialkyl-ethanolamin im Seitenabzug erhalten wird.

Verfahrensgemäß fällt das N,N-Dialkyl-ethanolamin der Formel II, z. B. DMEOA, in 99,00 - 99,99 Gew.-%iger, besonders 99,50 bis 99,99 Gew.-%iger, Reinheit an.

Im Besonderen liefert das Verfahren das N,N-Dialkyl-ethanolamin der Formel II mit einer APHA-Farbzahl ≤ 15, besonders ≤ 13, weiter besonders ≤ 11, z. B. im Bereich von 0 bis 10.

Die Spezifikation von im Verfahren eingesetztem EO lautet bevorzugt: > 99,9 Gew.-% EO, < 60 ppm H₂O, < 50 ppm Acetaldehyd, < 20 ppm CO₂, < 20 ppm Essigsäure.

Die Spezifikation von im Verfahren bevorzugt eingesetztem Dimethylamin (DMA) lautet im Besonderen: > 99,5 Gew.-% DMA, < 0,1 Gew.-% Monomethylamin (MMA), < 0,1 Gew.-% NH₃, < 0,1 Gew.-% MeOH, < 0,05 Gew.-% (Trimethylamin) TMA, < 0,2 Gew.-% H₂O.

ppm-Angaben beziehen sich auf die Masse (Massen-ppm).
Alle Druckangaben beziehen sich auf den Absolutdruck.
Die Ermittlung der APHA-Farbzahl erfolgt gemäß DIN-ISO 6271.

### Beispiele

### Beispiel 1

Mehrere Stahlfässer und mehrere mit dem High-Density-Polyethylen (HD-PE) Lupolen 5261 Z, erhalten von lyondellbasell, ausgekleidete Stahlfässer wurden mit N₂ zwei Min. inertisiert, so dass ein O₂-Restgehalt von 1,7 Vol.-% in der Atmosphäre verblieb. Die Fässer wurden mit DMEOA befüllt. Das DMEOA zeichnet sich dadurch aus, dass es in einer Fahrweise erhalten wurde, in der es bei seiner Herstellung aus EO und Dimethylamin in dem Synthesereaktor einer Verweilzeit von 3,7 Min. unterlag (Hochlastproduktion), durch die Nebenkomponenten wie z. B. Acetaldehyd-Vorläufer im DMEOA enthalten sind, die später Farbkomponenten bilden können. Es wurden zu den in der unteren Tabelle angegebenen Zeitpunkten von jedem Fasstyp je 2 Fässer geöffnet und davon je 2 Farbzahlproben gemessen. Die angegebenen Werte sind Durchschnittswerte aus je 2 x 2 gemessenen Werten. Zu jeder neuen Messung wurden Fässer geöffnet, die vorher noch nicht geöffnet waren. Die Hazen-Farbzahlen nach APHA wurden mit einem Gerät von Lange Lico 400 mit 50 mm Küvetten gemessen. Die zugrunde liegende Norm ist die ISO 6271. Die Lagerung erfolgte bei 20°C. Folgende Daten wurden gemessen:

| Lagerzeit in Tagen | Stahlfass | Kunststoff ausgekleidetes Stahlfass |
|---|---|---|
| 0 (vor Abfüllung) | 7 | 7 |
| 18 | 35 | 15 |
| 47 | 55 | 22 |
| 109 | 55 | 26 |
| 171 | 48 | 29 |

Man erkennt, dass die DMEOA Ware im Stahlfass schneller verfärbt, als im Kunststofffass.

### Beispiel 2

Zusätzlich zu den beiden in Beispiel 1 beschriebenen Fasstypen wurde für diesen Farbzahlsta-bilitätsversuch ein Kunststofffasstyp getestet, wobei dieser Behälter folgenden Coex-Aufbau (von außen nach innen) besitzt:
Außenschicht: HD-PE (Histif 5431 Z, erhältlich von lyondellbasell), schwarz eingefärbt mit leitfä-higem Russ,
Mittelschicht: HD-PE (Histif 5431 Z),
Innenschicht: HD-PE (Histif 5431 Z), natur, nicht eingefärbt, ohne leitfähigen Kontaktstreifen.

Die Fässer wurden analog Beispiel 1 zunächst inertisiert, dann mit DMEOA befüllt und die Farbzahlproben wurden analog Beispiel 1 vermessen. Die Lagerung erfolgte bei 20°C. Das DMEOA zeichnet sich dadurch aus, dass es in einer Fahrweise erhalten wurde, in der es bei seiner Herstellung aus EO und Dimethylamin in dem Synthesereaktor einer Verweilzeit von 3,7 Min. unterlag (Hochlastproduktion), durch die Nebenkomponenten wie z. B. Acetaldehyd-Vorläufer im DMEOA enthalten sind, die später Farbkomponenten bilden können. Die Hazen-Farbzahlen nach APHA waren jeweils vor der Abfüllung gleich 0.

Bei dem DMEOA, welches im Stahlfass gelagert wurde, wurde nach 121 Tagen eine Farbzahl von 21 APHA gemessen. Selbst bei längerer Lagerzeit von 275 Tagen wurde bei dem DMEOA, welches im Kunststoff ausgekleideten Stahlfass gelagert wurde, eine Farbzahl von 9 APHA gemessen. Bei dem DMEOA, welches im reinen Kunststofffass gelagert wurde, wurde nach 275 Tagen Lagerung eine Farbzahl von 7 APHA gemessen.

## Patentansprüche

1. Verfahren zur Verbesserung der Farbstabilität eines Ethanolamins, **dadurch gekennzeichnet, dass** das Ethanolamin in einem aus Kunststoff gefertigten Behälter gelagert wird.

2. Verfahren nach Anspruch 1, **dadurch gekennzeichnet, dass** es sich bei dem Behälter um ein Kunststofffass oder einen Kunststofftank handelt.

3. Verfahren nach Anspruch 1, **dadurch gekennzeichnet, dass** der aus Kunststoff gefertigte Behälter die Innenauskleidung eines Stahlfasses oder eines Stahltanks darstellt.

4. Verfahren nach einem der vorhergehenden Ansprüche, **dadurch gekennzeichnet, dass** es sich bei dem Kunststoff um ein Polyethylen, Polypropylen, Polystyrol, Polyurethan, Poly-tetrafluorethen, Polyketon, Polyamid, Epoxy-Harz, Epoxy-Amin-Harz, Phenolharz, einen Polyester, Polyvinylester oder Polyvinylether handelt.

5. Verfahren nach dem vorhergehenden Anspruch, **dadurch gekennzeichnet, dass** es sich bei dem Polyethylen-Kunststoff um ein HD-PE (High-Density-PE), LLD-PE (Linear-Low-Density-PE) oder LD-PE (Low-Density-PE) handelt.

6. Verfahren nach einem der vorhergehenden Ansprüche, **dadurch gekennzeichnet, dass** es sich bei dem Ethanolamin um Monoethanolamin (MEOA), Diethanolamin (DEOA), Triethanolamin (TEOA) oder ein N-Alkyl-diethanolamin der Formel I 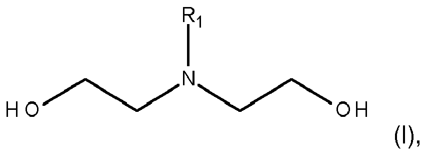 wobei R¹ eine C₁- bis C₈-Alkylgruppe bedeutet, handelt.

7. Verfahren nach einem der vorhergehenden Ansprüche 1 bis 5, **dadurch gekennzeichnet, dass** es sich bei dem Ethanolamin um ein N,N-Dialkyl-ethanolamin der Formel II 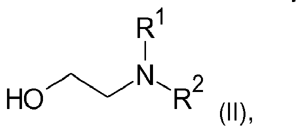 wobei R¹ und R² unabhängig voneinander eine C₁- bis C₈-Alkylgruppe bedeuten, handelt.

8. Verfahren nach dem vorhergehenden Anspruch, **dadurch gekennzeichnet, dass** es sich bei dem Ethanolamin um ein N,N-Dialkyl-ethanolamin der Formel II, wobei R¹ und R² unabhängig voneinander eine C₁- bis C₄-Alkylgruppe bedeuten, handelt.

9. Verfahren nach einem der beiden vorhergehenden Ansprüche, **dadurch gekennzeichnet, dass** es sich bei dem Ethanolamin um N,N-Dimethyl-ethanolamin (DMEOA) handelt.

10. Verfahren nach einem der vorhergehenden Ansprüche, **dadurch gekennzeichnet, dass** das Ethanolamin zuvor in einem oder mehreren Reaktoren durch Umsetzung von Ethyl-enoxid (EO) mit Ammoniak oder einem entsprechenden Monoalkylamin (R¹NH₂) oder einem entsprechenden Dialkylamin (R¹R²NH) hergestellt wurde.

11. Verfahren nach einem der Ansprüche 7 bis 9, **dadurch gekennzeichnet dass** das N,N-Dialkyl-ethanolamin der Formel II zuvor durch eine kontinuierliche Umsetzung von Ethylenoxid (EO) mit einem entsprechenden Dialkylamin (R¹R²NH) in Gegenwart von Wasser hergestellt wurde, wobei die Reaktionstemperatur im Bereich von 90 bis 180 °C und die Verweilzeit (VWZ) im Reaktor im Bereich von 1 bis 8 Min. lag, der Reaktoraustrag bei einer Temperatur im Bereich von 80 bis 160 °C über eine Zeit im Bereich von 20 bis 1000 Min. thermisch behandelt und danach das N,N-Dialkyl-ethanolamin destillativ abgetrennt wurde.

12. Verfahren nach dem vorhergehenden Anspruch, **dadurch gekennzeichnet, dass** die Edukte in einem molaren Verhältnis von Dialkylamin : EO im Bereich von 1,1 bis 10 eingesetzt wurden.

13. Verfahren nach einem der vorhergehenden Ansprüche, **dadurch gekennzeichnet, dass** das betreffende Ethanolamin bei seiner Herstellung in dem bzw. den Synthesereaktoren insgesamt einer Verweilzeit von ≤ 5 Min. unterlag.

14. Verfahren nach einem der vorhergehenden Ansprüche zur Herstellung eines Ethanola-mins mit einer APHA-Farbzahl ≤ 35 nach einer Lagerzeit von 25 Wochen bei 20°C, wobei die APHA-Farbzahl zu Beginn ≤ 15 betrug.

15. Verfahren nach einem der vorhergehenden Ansprüche, **dadurch gekennzeichnet, dass** die Lagerung in Abwesenheit von Sauerstoff erfolgt.

16. Verwendung eines aus Kunststoff gefertigten Behälters nach einem der Ansprüche 1 bis 5 zur farbstabileren Lagerung eines Ethanolamins gemäß einem der Ansprüche 6 bis 15.
